# EUROPEAN PATENT APPLICATION

(11) **EP 1 153 612 A1**
(43) Date of publication of application: **14.11.2001**
(21) Application number: 00902902.6
(22) Date of filing: 10.02.2000
(51) Int. Cl.: A61K 45/00, A61P 35/00

(54) **ANTICANCER AGENT POTENTIATORS**

(30) Priority: 10.02.1999 JP 3300899
(71) Applicant: Tsuruo, Takashi, Tokyo 156-0051 (US); THE KITASATO INSTITUTE, Tokyo 108-8642 (JP); Nippon Kayaku Kabushiki Kaisha, Tokyo 102-8172 (JP)
(72) Inventor: Tsuruo, Takashi, Tokyo 156-0051 (JP); Tomida, Akihiro, Tokyo 112-0002 (JP); Ogiso, Yasunari, Tokyo 113-0022 (JP); Omura, Satoshi, Minato-ku, Tokyo 108-8642 (JP)
(74) Representative: Ritter, Stephen David
(86) International application number: JP0000741
(87) International publication number: WO0047230

(57) **Abstract**

Using a proteasome activity inhibitor in combination with an anticancer drug using a topoisomerase activity inhibitor inhibits proteasome activity progressing degradation of topoisomerase to keep an enough amount of topoisomerase in cancer cells and can enhance the effect of the anticancer drug using the topoisomerase activity inhibitor targeting this. On the other hand, similarly, combined use with the proteasome activity inhibitor can enhance the effect of a DNA strand-damaging type anticancer drug directly acting to DNA, such as platinum complexes, alkylating agents, and bleomycins, whose effect is suppressed or reduced by the proteasome activity.

## Description

### TECHNICAL FIELD

The present invention relates to an enhancer enhancing an effect of an anticancer drug, which is a drug set combining the enhancer with the anticancer drug, and the like.

### BACKGROUND ART

Cancer therapy is mainly classified into therapies mainly using a surgical treatment and treatment using radiation and the like and chemotherapies using anticancer drugs and usually conducted using a combination of two or more treatments.

Chemotherapy is mainly conducted by administration of anticancer drugs. In accordance with a progress of research on various substances and physiological functions relating to oncogenesis and proliferation of cancer cells and progression of a tumor, anticancer drugs using various functions have been commercialized.

Such anticancer drug is exemplified by a topoisomerase inhibitor.

Topoisomerases are enzymes that change DNA conformation through breakage and reunion and found in a wide range of biological species. Two types of topoisomerase, i. e., type I and type II, have been known. The type II is divided into type α and type β. The type I acts on a single strand of DNA and the type II acts on a double strand of DNA. These topoisomerases are important enzymes relating to replication and transcription of DNA. If inhibition of the activity of this enzyme stabilizes a DNA-enzyme complex produced in a catalytic reaction step to left it as it is, such disturbance as that the DNA is left in a cleaved state occurs. As a result, cellular activities are disturbed to make some cells die. Topoisomerase inhibitors are considered to exert their effects as anticancer drugs due to the inhibition effect.

As anticancer drugs having the function as the topoisomerase inhibitor, etoposide (VP-16), camptothecin and its derivative (for example, CPT-11), and the like have been known. Etoposide (VP-16) exerts the effect as an anticancer drug using the function as a topoisomerase II activity inhibitor and has already been used as a standard therapeutics agent of multiple drug-combined chemotherapy against testicular tumor and small cell lung cancer. In addition, application thereof has been extended to malignant lymphoma, acute leukemia, non-small cell lung cancer, urinary bladder cancer, stomach cancer, and the like. On the other hand, CPT-11 is a camptothecin derivative and exerts the effect as an anticancer drug using the function as a topoisomerase I activity inhibitor. Phase II clinicals trial of CPT-11 used alone against various cancers were conducted and it was found to be effective for small cell lung cancer, non-small cell lung cancer, colon cancer, ovarian cancer, cervical carcinoma, stomach cancer, malignant lymphoma, and the like.

On the other hand, platinum complexes such as cisplatin, alkylating agents such as mitomycins, and bleomycins such as bleomycin are regarded as anticancer drugs exerting the effect by acting directly to DNA of a cancer cell to damage a DNA strand.

A major problem of cancer therapy using anticancer drugs is that cancer cells acquire resistance to the anticancer drugs to cause a decreasing and disappearance of the effect of the anticancer drugs. For example, acquisition of multiple drug resistance by cancer cells through expression of a large quantity of P-glycoprotein of a representative example of development of resistance.

With respect to the anticancer drugs using topoisomerase activity inhibitors described above, in accordance with kind of cancer, for example, an antitumor effect is exhibited against various solid cancers and cure may be attained in a few occasions. And, even for a cancer to which these anticancer drugs are indicated, it is frequently experienced to have almost no effect from an initial stage of therapy. In addition, although these anticancer drugs are applied to a relatively wide range of solid cancers, these are not therapeutically effective and not indicated for many cancers. In other words , despite that these anticancer drugs have an excellent antitumor effect against various solid cancers, a sufficient therapeutic effect is not attained due to drug resistance intrinsic to solid cancers in many cases. For example, the following cases have been known that for solid cancers having a small size of the tumor, the anticancer drug is effective, but for a large tumor, the action of the anticancer drug lowers or the effect disappears. It is being proven that particular environment such as glucose starvation and hypoxia created inside the solid cancer due to insufficient vascularization is involved in such drug resistance of solid cancers.

### Disclosure of the Invention

The present invention includes the following aspects .
1. An anticancer drug enhancer, to enhance an effect of an anticancer drug, characterized in that an effective ingredient is a substance inhibiting an activity of proteasome having a function to enhance an effect of a DNA strand-damaging type anticancer drug to cancer cells.
2. The anticancer drug enhancer according to the above described aspect 1, characterized in that the above described DNA strand-damaging type anticancer drug is an anticancer drug whose effect is suppressed or reduced in the presence of an activity of proteasome.
3. The anticancer drug enhancer according to the above described aspect 1, characterized in that the above described anticancer drug contains a substance inhibiting an activity of topoisomerase as the effective ingredient.
4. The anticancer drug enhancer according to the above described aspect 2, characterized in that the anticancer drug is at least one of a substance inhibiting an activity of topoisomerase I and a substance inhibiting an activity of topoisomerase II.
5. The anticancer drug enhancer according to the above described aspect 1, characterized in that the above described DNA strand-damaging type anticancer drug is an anticancer drug damaging DNA strand by acting directly to the DNA strand.
6. The anticancer drug enhancer according to any one of the above described aspect 1 to aspect 5, characterized in that the above described substance inhibiting the activity of proteasome is a selective proteasome activity inhibiting agent.
7. The anticancer drug enhancer according to any one of the above described aspect 1 to aspect 5, characterized in that the above described substance inhibiting the activity of proteasome is a non-peptide aldehyde proteasome activity inhibiting agent.
8. The anticancer drug enhancer according to any one of the above described aspect 1 to aspect 7, characterized in that the above described cancer is a solid cancer.
9. The anticancer drug enhancer according to any one of the above described aspect 1 to aspect 7, characterized in that the above described cancer is under a physiological stress.
10. The anticancer drug enhancer according to the above described aspect 9, characterized in that the above described physiological stress is glucose starvation stress or hypoxia stress.
11. A drug set of an anticancer drug and an enhancer to enhance an effect of the anticancer drug, characterized in that the above described enhancer is a substance inhibiting an activity of proteasome which has a function to enhance the effect of the above described anticancer drug by inhibiting the activity of proteasome in a cancer cell and
   the above described anticancer drug is an anticancer drug whose effect can be suppressed or reduced in the presence of the proteasome activity.
12. The drug set according to the above described aspect 11, characterized in that the above described anticancer drug contains the substance inhibiting the activity of topoisomerase as the effective ingredient.
13. The drug set according to the above described aspect 12, characterized in that the anticancer drug is at least one of a substance inhibiting an activity of topoisomerase I and a substance inhibiting an activity of topoisomerase II.
14. The drug set according to the above described aspect 11, characterized in that the above described substance inhibiting an activity of proteasome is lactacystin.
15. The drug set according to any one of the above described aspect 11 to aspect 14, being a drug set of 2 preparations comprising a preparation containing the above described anticancer drug and a preparation containing other above described substance inhibiting an activity of proteasome.
16. The drug set according to any one of the above described aspect 11 to aspect 14, having a mixture form containing the above described anticancer drug and the above described substance inhibiting the activity of proteasome in a same preparation.
17. The drug set according to any one of the above described aspect 11 to aspect 16, characterized in that the above described cancer is a solid cancer.
18. The drug set according to any one of the above described aspect 11 to aspect 16, characterized in that the above described cancer is under physiological stress.
19. The drug set according to the above described aspect 18, characterized in that the above described physiological stress is glucose starvation stress or hypoxia stress.
20. A drug set of an anticancer drug damaging DNA by acting directly to the DNA and an agent inhibiting the activity of proteasome.
21. The drug set according to the above described aspect 20, characterized in that the anticancer drug damaging DNA by acting directly to the DNA is platinum complexes, alkylating agents, and bleomycins.
22. The drug set according to the above described aspect 20, characterized in that the substance inhibiting an activity of proteasome is a selective proteasome activity inhibiting agent.
23. The drug set according to the above described aspect 22, characterized in that the selective proteasome activity inhibiting agent is lactacystin.
24. A therapeutic method for cancer characterized in that a DNA strand-damaging type anticancer drug and a substance inhibiting the activity of proteasome that has a function to enhance the effect thereof against cancer cells, is administered.
25. Use of a substance inhibiting an activity of proteasome and having a function to enhance an effect of a DNA strand-damaging type anticancer agent to cancer cells for manufacturing an agent to enhance the effect of the anticancer drug.

The present invention was created in consideration of a subject in a range of indication of the above described topoisomerase inhibitor as an anticancer drug and a problem of development of resistance of cancer cells, particularly in solid cancers. The object of the present invention is to provide a technique to make more effective prevention and treatment of cancers possible through an increase in range of indication of the topoisomerase inhibitor as an anticancer drug and enhancement of the effect thereof and also, to provide a technique to inhibit development of resistance of cancer cells, particularly development of resistance of solid cancers to allow to exert effects of the topoisomerase inhibitor and the like more effectively as an anticancer drug.

Such an object can be achieved according to the respective aspects of the present invention as described above.

In the case where the agent to enhance the effect of the anticancer drug according to the present invention is used in combination with an anticancer drug containing the topoisomerase activity inhibiting agent as an effective ingredient, by action of this to cancer cells, for example, under a physiological stress, of which topoisomerase activity has been increased, an enough quantity of topoisomerase itself is held by cancer cells to inhibit reunion after cleavage of DNA by this, resulting in cleavage, inappropriate for cancer cells, of DNA, a cell activity is lowered, cells are killed, and thus, the therapy by using an effective anticancer drug becomes possible.

In the case where the agent to enhance the effect of the anticancer drug according to the present invention is used in combination with an anticancer drug damaging a DNA strand by acting directly to the DNA, the effect of the present invention is expressed. In the present status, where an interaction of the anticancer drug damaging the DNA strand by acting directly to the DNA with proteasome has not been known, it is an unexpected result that the proteasome inhibiting agent has such an action to enhance the effect of the anticancer drug.

### BRIEF DESCRIPTION OF THE DRAWINGS

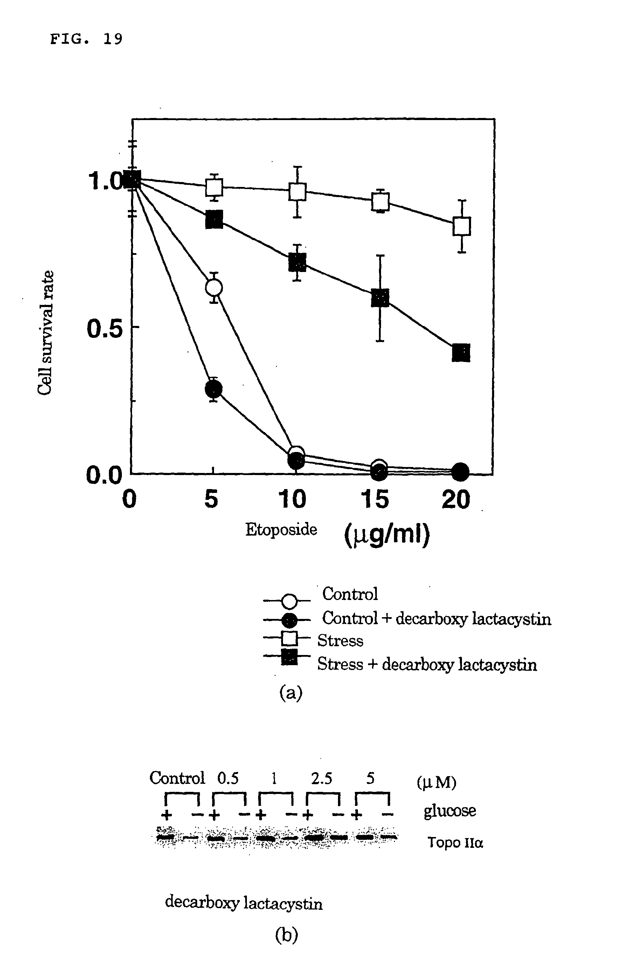

FIG. 1 is a figure showing to sensitivity to etoposide of cancer cells in a glucose starvation condition and an effect of PSI thereon, observed in Example 1;
FIG. 2 is a figure showing the effect of each inhibiting agent on etoposide sensitivity in the glucose starvation condition observed in Example 1;
FIG. 3 is a figure showing the effect of lactacystin on etoposide sensitivity in the glucose starvation condition of cancer cells, observed in Example 1;
FIG. 4 is a figure showing the effect of lactacystin on Doxorubicin sensitivity in the glucose starvation condition of cancer cells, observed in Example 1;
FIG. 5 is a figure showing the result of western blotting to determine expression of topoisomerase IIα observed in Example 2. (a) shows the effect of PSI, MG132, and MG115 in the glucose starvation condition. (b) shows the effect of E64 and ZLLal in the glucose starvation condition. (c) shows the effect of lactacystin in the glucose starvation condition. (d) shows the effect of PSI, MG132. and MG115 in hypoxia condition. (e) shows the effect of E64 and ZLLal in hypoxia condition;
FIG. 6 is a photomicrograph to replace to a drawing of the cells, showing the result of cell staining in Example 3;
FIG. 7 is a figure showing the result of western blotting in Examples 3, (a) and (b) show contents of P27 and P32, respectively, in the glucose starvation condition and (c) and (d) show contents of P27 and P32, respectively, in the hypoxia condition in solution extracted from nuclear and whole cells;
FIG. 8 is a figure showing the result of measurement of proteasome activity of the solution extracted from the nuclei of cells of a control and in the glucose starvation condition, observed in Example 4. (a) shows the result when Z-LLL-MCA as a substrate was used and (b) shows the result of Suc-LLVY-MCA as the substrate;
FIG. 9 is a figure showing the result of measurement of proteasome activity of the solution extracted from nuclei of cells of the control and in the hypoxia condition, observed in Example 4; (a) shows the result when Z-LLL-MCA was used as a substrate and (b) shows the result of Suc-LLVY-MCA as a substrate;
FIG. 10 is a figure showing the effect of each inhibiting agent against proteasome activity of the solution extracted from the nuclei, observed in Example 4; (a) shows the result when the solution extracted from the nuclei of cells of the control and under the glucose starvation condition and (b) shows the result when the solution extracted from the nuclei of the cell of control and under hypoxia condition;
FIG. 11 is a figure showing the therapeutic result of combined use of lactacystin and etoposide as an anticancer drug in a mouse line to which a cancer cell was transplanted, observed in Example 5;
FIG. 12 is a figure showing the therapeutic result of combined use of lactacystin and etoposide as an anticancer drug in a mouse line to which a cancer cell was transplanted, observed in Example 6;
FIG. 13 is a figure showing the therapeutic result of combined use of lactacystin and Doxorubicin as an anticancer drug in a mouse line to which a cancer cell was transplanted, observed in Example 7;
FIG. 14 is a figure showing a change in a cell-killing effect of combined use of lactacystin and camptothecin targeting topoisomerase I, observed in Example 8;
FIG. 15 is a figure showing a change of a cell-killing effect in combined use of PSI and camptothecin targeting topoisomerase I, observed in Example 8;
FIG. 16 is a figure showing a change of a cell-killing effect in combined use of MG132 and camptothecin targeting topoisomerase I, observed in Example 8;
FIG. 17 is a figure showing the result of western blotting in Example 8, (a) shows an expression of topoisomerase I in the glucose starvation condition and the effect of clasto-lactacystin β lactone thereto, and (b) shows those of topoisomerase IIα;
FIG. 18 is a figure showing a change of a cell-killing effect in combined use of lactacystin and cisplatin observed in Example 9;
   The top (a) of FIG. 19 is a figure showing a change of the cell-killing effect in combined use of decarboxy lactacystin yielded from Example 10 with etoposide, which is an anticancer drug targeting topoisomerase II. The bottom (b) is a figure showing the result of western blotting observed in Example 10 and also shows the expression of topoisomerase IIα under the glucose starvation condition and the effect of decarboxy lactacystin thereto;

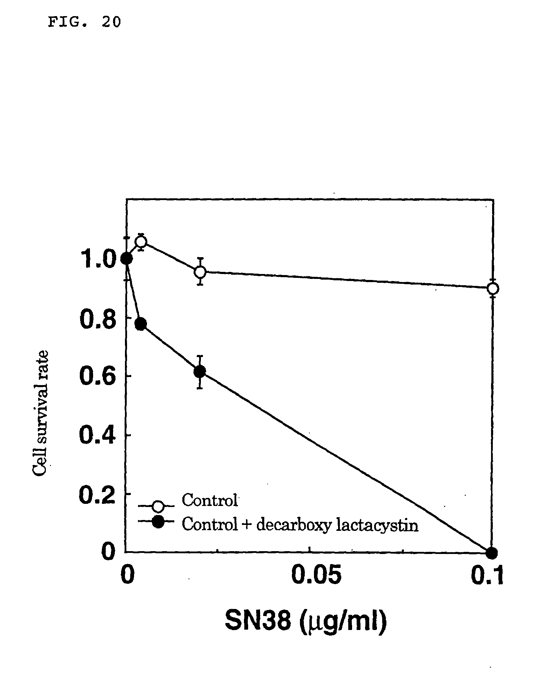

FIG. 20 is a figure showing the change of cell-killing effect in combined use of decarboxy lactacystin yielded from Example 11 and SN38, which is an anticancer drug targeting topoisomerase I; and

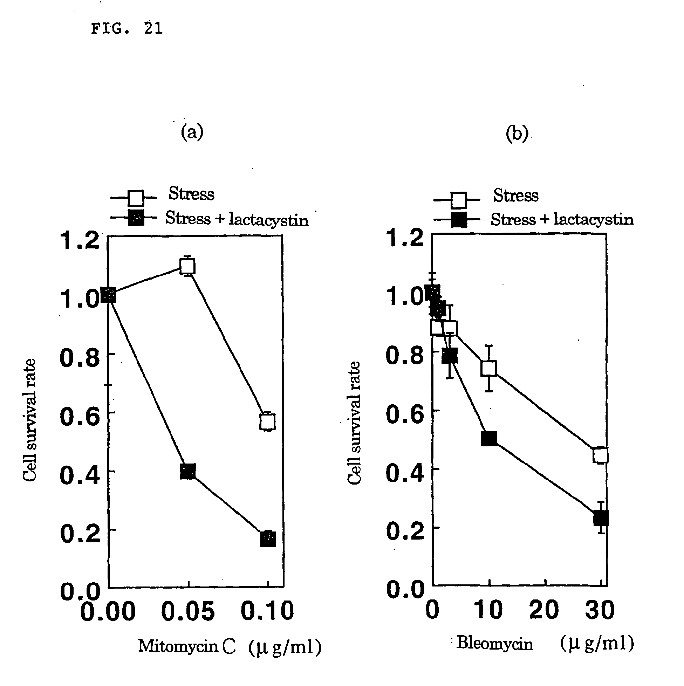

FIG. 21 is a figure showing the change of cell-killing effect in combined use of mitomycin C and bleomycin with lactacystin in the cultured cell line obtained in Example 12.

### BEST MODE FOR CARRYING OUT THE INVENTION

During a study on a relation between the effect of the anticancer drug, whose effective ingredient is a topoisomerase inhibitor, against cancer cells and inhibition of proteasome activity, the present invention was created on the basis of the new finding that a combined use of a substance inhibiting the activity of proteasome with an anticancer drug of a DNA strand-damaging type allows to enhance the action of the anticancer drug.

Proteasome is a multifunctional high molecular weight protein complex found from a cytoplasm fraction and having a protein degradation activity and its role in cells has been insufficiently known. Many studies were carried out to reveal the role. As a function of proteasome, possibilities were pointed out relating various functions and phenomena of cells such as a cell cycle, apoptosis, signal transduction, metabolism, immune response, and the like. However, such proteasome is not a simple protease, but has multifunctionality and thus, in the case where proteasome activity, particularly the activity in cancer cells, is inhibited, what change of cell functions occurs, as a result, what phenomenon occurs in cancer cells, and what change occurs in sensitivity to the anticancer drug cannot be presumed on the basis of studies so far carried out, therefore, enhancement of the anticancer effect by inhibition of the proteasome activity in the enhancer for the anticancer effect in the present invention cannot be deduced from the conventional art.

Specific examples of anticancer drug used in combination with the enhancer for the anticancer effect in the present invention are not specially restricted.

The anticancer drug of the DNA strand-damaging type directly or indirectly damaging the DNA strand is preferable. The anticancer drug of the DNA strand-damaging type directly damaging with the DNA strand is the anticancer drug, that is understood as has the function to inhibit DNA synthesis and following cell division of cancer cells by direct action to the DNA strand in cancer cells and exemplified by platinum complex, alkylating agents, and bleomycins. Platinum complex is exemplified by cisplatin and carboplatin. Alkylating agents are exemplified by mitomycin, cyclophosphamide, Iphosphamide, Busulfan, thiotepa, melphalan, and the like. Bleomycins are exemplified by bleomycin, Peplomycin, and the like.

The anticancer drug of the DNA strand-damaging type indirectly damaging the DNA strand does not act directly to DNA, but the anticancer drug having an action to damage indirectly the DNA strand through the enzyme involving in cleavage and reuniting of the DNA. Such anticancer drug is exemplified by topoisomerase inhibitor. The topoisomerase inhibitor has the action to inhibit the activity of topoisomerase involving in cleavage and reunion of DNA and hence, inhibit cleavage and reuniting of DNA and cause a disorder of cell activity resulting in death of cancer cells.

Topoisomerase used as the target in case using the topoisomerase inhibitor is exemplified by topoisomerase I and topoisomerase II (IIα and IIβ).

The inhibiting agent of topoisomerase I is exemplified by camptothecin and its derivative (for example, Irinotecan (CPT-11), Topotecan, 9-nitrocamptothecin, 9-aminbcampotothesin, 10, 11-methylene dioxy camptothecin, DX-8951F, GG-211, and the like), and the like. The inhibiting agent of topoisomerase II is exemplified by etoposide, doxorubicin, teniposide, daunomycin, actinomycin D, mitoxantron, m-AMSA, 2-methyl-9-OH-ellipticinium acetate, and the like. In addition, TAS-103 expressed by the following structural formula can be exemplified as that inhibiting both topoisomerase I and II.

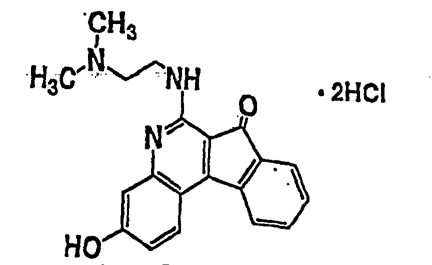

The agent to enhance the effect of the anticancer drug according to the present invention can be preferably used for the anticancer drug, whose action as an anticancer drug is suppressed or lowered in the presence of the activity of proteasome, as the target of resistance acquisition in which proteasome involved. And, by inhibiting the activity of proteasome, a possibility to develop an applicable range of the anticancer drug, whose effect has not been confirmed so far, to a new cancer species is expanded largely.

In case using the topoisomerase inhibitor as an anticancer drug, the effect is considered to be exerted by inhibiting the activity of proteasome and providing an environment where the topoisomerase inhibitor, that is the anticancer drug targeting topoisomerase, can effectively act in cancer cells.

As the agent to enhance the effect of the anticancer drug according to the present invention, one having the effect against cancer cells, particularly cancer cells under the physiological stress in which the activity of proteasome inside a nucleus has risen twice or three times a normal value, is preferable. Particularly, one effective against an internal cancer cell with a distance from a blood vessel of a solid cancer (solid tumor) under such stress, is more preferable.

The agent to enhance the effect of the anticancer drug according to the present invention contains the proteasome inhibitor as the effective ingredient to inhibit the activity of proteasome. The substance to inhibit the activity of proteasome may, on the basis of accumulation of proteasome in the nuclei of cancer cells in a state under the physiological stress, be the substance not removed by cancer cells to make reach inside of the nuclei possible, effectively inhibitable of proteasome activity in the nuclei, and allowable pharmacologically. Such substances are, for example, exemplified by PSI (carbobenzoxy-L-Isoleucyl-γ-t-Butyl-L-Glutamyl-L-Alany 1-L-Leucinal (Peptide Institute, Inc.), MG115 (carbobenzoxy-L-Leucyl-L-Leucyl-L-Norvalinal: Peptide Institute, Inc.), MG132
(carbobenzoxy-L-Leucyl-L-Leucyl-Leucinal: Peptide Institute, Inc.), lactacystin (The Kitasato Institute). Using 1 or more of these as the effective ingredient, the agent to enhance the effect of the anticancer drug can be prepared. Among these, lactacystin being non-peptide compound is specific in inhibition of proteasome activity and therefore, more preferable.

The above described PSI, MG115, and MG132 show inhibitory activity on other enzymes such as calpain and show low selectivity as the proteasome inhibiting agent. On the other hand, for example, lactacystins show no inhibitory activity on calpain and can be said as a selective proteasome activity inhibitor. According to the present invention, in consideration of adverse effects and effects on an animal, a selective proteasome activity inhibitor is more preferable. In contrast to the above described 3 compounds (PSI, MG115, andMG132) that are peptides having an aldehyde group, lactacystins have no such aldehyde group and can be said as a non-peptide aldehyde proteasome activity inhibiting agent.

For reference, lactacystin has been described in Japanese Patent Laid-Open No. 3-98594 and Japanese Patent Laid-Open No. 8-231501, and also J. Antibiotics 19 (44): 113-116, 1991.

In addition, for derivatives of these proteasome inhibiting agent, in the range to keep the function as the agent to enhance the effect of the anticancer drug through the proteasome inhibiting effect, derivatives the molecular structure of which has been changed by such as modification with a substitution group can be preferably used in the present invention. Derivatives of lactacystin can be, for example, exemplified by
des-N-acetylaminolactacystin (refer to Japanese Patent Laid-Open No. 8-321501), in which R in the following structural formula (1) is-SCH₂CH₂COOH and decarboxy lactacystin (refer to J. Antibiotics 48 (4) : 747-748, 1995) of which R is-SCH₂CH₂NHCOCH₃ 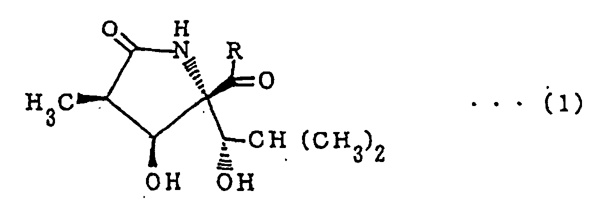 and clasto-lactacystin β lactone of the following structural formula, and the like.

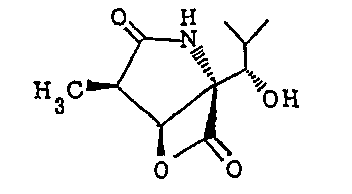

The two derivatives expressed by the above described structural formula (1) has been disclosed in the above described Japanese Patent Laid-Open No. 8-321501.

The agent for enhancing the anticancer drug according to the present invention can be, when required, prepared as a drug by blending, when required, the proteasome activity-inhibitor, which has the above described characteristics, with a pharmaceutically acceptable carrier and diluting agent.

A form of preparation can be exemplified by a solid form such as tablet, pill, powder, granule, capsule, suppository, and the like, a liquid form such as injection, suspension, syrup, emulsion, and the like, a semisolid form such as plaster and drug preparation can be carried out by properly selecting the supporter, diluting agent, vehicle, and various kinds of additives in accordance with these dosage forms. An amount the proteasome activity-inhibitor in the drug may be properly selected on the basis of dosage of an anticancer drug following a therapeutic plan according to a symptom of a patient who is an object of the therapy. For example, 1 dose can range from about 20 to 100 mg; however, it is not restricted to this.

The dosage of this enhancer to the patient can be selected on the basis of the amount of administration of the anticancer drug following the therapeutic plan. For example, about 100 to 500 mg a day is possible; however, it is not restricted to this. In addition, an administration timing is set to allow an effective concentration of the anticancer drug in cancer cells in the state, where the proteasome activity is effectively inhibited. For example, in case using the topoisomerase inhibitor as the anticancer drug, in the state where degradation of topoisomerase by proteasome has been suppressed, setting is better to carry out to yield the effective concentration of the anticancer drug in cancer cells. An adoptable form of administration can, for example, be exemplified by a method in which in administration of the anticancer drug, according to the present invention with a periodical interval, the agent for enhancing the effect of the anticancer drug is administered before administration of the anticancer drug with a proper time interval; the method in which the anticancer drug is simultaneously administered with the agent for enhancing the effect of anticancer drug and these dosage forms are selected to make a time difference in time of absorption by these patients; or, these are simultaneously administered to keep concentration in a body of the patient to a predetermined concentration level; and the like.

On the other hand, as the anticancer drug, those. whose action as the anticancer drug is influenced by suppression or decrease in the presence of the proteasome activity, can be used in combination with the agent for enhancing the effect of the anticancer drug according to the present invention.

For reference, as the anticancer drug, at least 1 species, which expresses the effect in combination with the anticancer drug enhancer according to the present invention is used and when required, other anticancer drug effective in combined use may be used in combination. The anticancer drug other than this may be one whose effect is enhanced by the agent for enhancing the effect of the anticancer drug in the present invention and may be one presents no enhancement.

In case using the topoisomerase inhibitor, the anticancer drug can be prepared using 1 or more species thereof as the effective ingredient. In this case, the agent for enhancing the effect of the anticancer drug in the present invention may be one that enhances at least any one of the effect as the anticancer drug of the topoisomerase I inhibitor and the effect as the anticancer drug of the topoisomerase II inhibitor; one enhancing the effect of both these anticancer drugs is more preferable.

The anticancer drug includes derivatives of which molecular structure has been changed by modification by a substitution group in the range keeping the effect thereof as the anticancer drug and the effect of enhancing the effect of the anticancer drug in combined use with the proteasome activity-inhibitor.

The form of the anticancer drug can be various drug forms in the above described agent for enhancing the effect of the anticancer drug and the anticancer drug marketed and one prepared in the drug form using these effective ingredients can be used. Therefore, the amount of the effective ingredient to be compounded in the drug preparation can be set as normally carried out. Combined use with the agent for enhancing the effect of the anticancer drug according to the present invention enhances the effect thereof and thus, it may be possible to make the amount compounded in the drug preparation lower than the case of no combined use.

In addition, the anticancer drug and the agent for enhancing the effect of the anticancer drug can be provided to a therapeutic site by setting the as 1 set. In this occasion, these drug preparations may be respectively supplied as alone separate drug preparation and also may be supplied as mixture by containing both of these components in a same drug preparation.

For example, for simultaneous administration of the anticancer drug and the agent for enhancing the effect of the anticancer drug, the method in which these prepared in different drug preparations are simultaneously administered and the method these contained in 1 drug preparation are supplied as mixture and these effective ingredients are administered to (absorbed in) the patient can be applied. Or, a composition of each protective of the anticancer drug and the agent for enhancing the effect of the anticancer drug is changed after a publicly known art to make a break-down period of the anticancer drug in a digestive organ system including, for example, stomach, intestine, and colon different from the break-down period of the agent for enhancing the effect of the anticancer drug, resulting in creation of difference in the period of absorption. For reference, the method for administration is not restricted to this and can be modified to achieve the effect of the present invention.

The agent for enhancing the effect of the anticancer drug according to the present invention is effective to the cancer to which the anticancer drug influenced by suppression of and decrease in the effect as the anticancer drug in the presence of the proteasome activity can be applied. In addition, in the cancer for which the effect of the anticancer drug combined with the agent for enhancing the effect of the anticancer drug according to the present invention has not been confirmed, if it is the cancer for which expression of resistance thereto by the proteasome activity, it can be the object of the therapy.

In the case combining with the anticancer drug of which effective ingredient is the topoisomerase activity inhibitor, for example, in therapy of testicular tumor, small cell lung cancer, malignant lymphoma, acute leukemia, non-small cell lung cancer, urinary bladder cancer, stomach cancer, colon cancer, ovarian cancer, cervical carcinoma, and the like, combined use with the anticancer drug is effective. On the other hand, it is effective to the cancer to which combined use of the topoisomerase activity inhibitor with other anticancer drug is applied. In addition, in the cancer for which the effect of the anticancer drug, of which effective ingredient is the topoisomerase activity inhibitor, is low or not observed, in the case where the proteasome activity inhibits this effect of the anticancer drug, the agent for enhancing the effect of the anticancer drug according to the present invention is effective. In other words, in the case where when the anticancer drug, of which effective ingredient is the topoisomerase activity inhibitor and the like express the effect thereof, a targeted enzyme (or, enzyme constructing an enzyme system) is degraded by proteasome to lose the effect of the anticancer drug and the like case, the agent for enhancing the effect of the anticancer drug according to the present invention is effective.

In addition, cancer cells under the physiological stress, for example, stresses of the glucose starvation condition and hypoxia condition have the intranuclear proteasome activity twice to three times higher than the normal condition and shows increase in degradation of topoisomerase and the like and hence, the agent for enhancing the effect of the anticancer drug according to the present invention is preferable to such cancer. On the other hand, to cells inside a tumor of the solid cancer, for example, cells in a position with a distance from a blood vessel inside a tumor lump enlarged in accordance with proliferation of cancer cells, the component such as nutrients and oxygen necessary for proliferation and maintenance of cells is insufficiently supplied and thus, the starvation condition of these components should appear. Consequently, for the solid cancer in such starvation condition, the agent for enhancing the effect of the anticancer drug according to the present invention is very effective; for other cancer, it is similarly effective to cancers in the state where the starvation condition has been artificially caused in a lesion of the patient.

In addition, in case combining with anticancer drugs, for example, platinum complexes the effect can be expected for cancers such as cisplatin, alkylating agents such as mitomycin, bleomycins such as bleomycin, and the like, expressing the effect by damaging the DNA strand through the direct action to the DNA of cancer cells, testicular tumor, urinary bladder cancer, pelvis of kidney-ureter tumor, prostate cancer, ovarian cancer, head-neck cancer, non-small cell cancer, esophagus cancer, cervical cancer, neuroblast tumor, stomach cancer, lymphatic leukemia, tonic mallow leukemia, colon rectum cancer, lung cancer, spleen cancer, hepatic cancer, cancer of the uterine body, breast cancer, skin cancer, malignant lymphoma, glioma, goiter, and the like according to the kind of the anticancer used.

### (Examples)

The present invention will be further described below in detail with reference to examples. Where, "%" means "% by weight" unless otherwise specially designated and "% v/v" means volume %.

### Example 1

(Enhancement of effect of the topoisomerase II-inhibiting anticancer drug in a cultured cell line)

In the glucose starvation environment found in the solid cancer, the effect of combined use of the proteasome inhibitor in a human colon adenocarcinoma cell HT-29 with the anticancer drug targeting topoisomerase II was tested.

Concerning culture media, for a culture condition in a normal state, RPMI 1640 medium (Nissui Pharmaceutical Co.-made) to which a 5% FBS (fetal bovine serum) was added and for the culture condition in the glucose starvation state symbolically found in the solid cancer, RPMI 1640 medium (Lifetech Oriental-made), lacking glucose, in which the 5% FBS was added were used, respectively. Culture was carried out using a CO₂ incubator (Tabai Espec-made) under conditions of 5% CO₂/95% air and humidity 100%.

In order to test sensitivity to the anticancer drug, the cell cycle synchronization culture methodwas employed. Nocodazol (Sigma made) was used to synchronize the cell cycle, cells in a logarithmic proliferation phase were treated with 40 ng/ml Nocodazol for 9 hours, and cells accumulated in cell division phase (Mpahse) were collected by gentle pipetting. M phase-synchronized cells collected through these steps were washed with PBS (phosphate buffered saline) followed by inoculation of 1×10⁵ cells per well of 12 well-culture plate.

M phase-synchronized cells were cultured under the normal condition and the glucose starvation condition to conduct treatments with the proteasome inhibiting agent and the anticancer drug as follows. For cells under the glucose starvation condition, the culture medium, to which the proteasome inhibiting agent and the protease inhibiting agent not inhibiting proteasome were added as the control group, and the culture medium, to which no inhibiting agent has been added, was used. For cells under the normal condition, the culture medium, to which each inhibiting agent was not added, was used. After inoculation of cells, culture was carried out for 13 hours in each culture medium. Subsequently, etoposide (Bristol Myerss Squibb K.K. made) and doxorubicin (Kyowa Hakko made) which are anticancer drugs targeting topoisomerase II were added to each culture medium and the cells were further cultured for 1 hour.

Sensitivity of cells to the anticancer drug was measured using the colony formation method. As described above, cells treated with the proteasome inhibiting agent and the anticancer drug were washed with PBS followed by soaking in trypsin (Lifetech Oriental made) solution and removed from a culture dish to suspend in an ordinary culture medium. These cells were inoculated again in 500 to 5000 cells for a 10 cm culture dish and cultured for 10 to 12 days to form a colony. The colony formed was fixed by 10% formaldehyde solution and stainedwith 0.01% crystal violet (Wako Pure Chemicals made) solution. Number of colony was counted and a Cell survival rate was calculated assuming that the number of colonies untreated with the anticancer drug is 1.

For reference, the proteasome inhibiting agent and the protease inhibiting agent not inhibiting proteasome used as described above were as follows.
(1) Proteasome inhibiting agent
   . PSI
      (carbobenzoxy-L-Isoleucyl-γ-t-Butyl-L-Glutamyl-L-Alany 1-L-Leucinal): final concentration 2.5 µM (Peptide Institute Inc. made)
   . MG115 (carbobenzoxy-L-Leucyl-L-Leucyl-L-Norvalinal): final concentration 2.5 µM (Peptide Institute Inc. made)
   . MG132 (carbobenzoxy-L-Leucyl-L-Leucyl-Leucinal) : final concentration 2.5 µM (Peptide Institute Inc. made)
   . Lactacystin, final concentration 10 µM (Kindly supplied from The Kitasato Institute made)
(2) Protease inhibiting agent not inhibiting proteasome
   . E64, final concentration 25 µM (Peptide Institute Inc. made)
   . ZLLal (carbobenzoxy-L-Leucyl-L-Leucinal): final concentration 25 µM (Peptide Institute Inc. made)

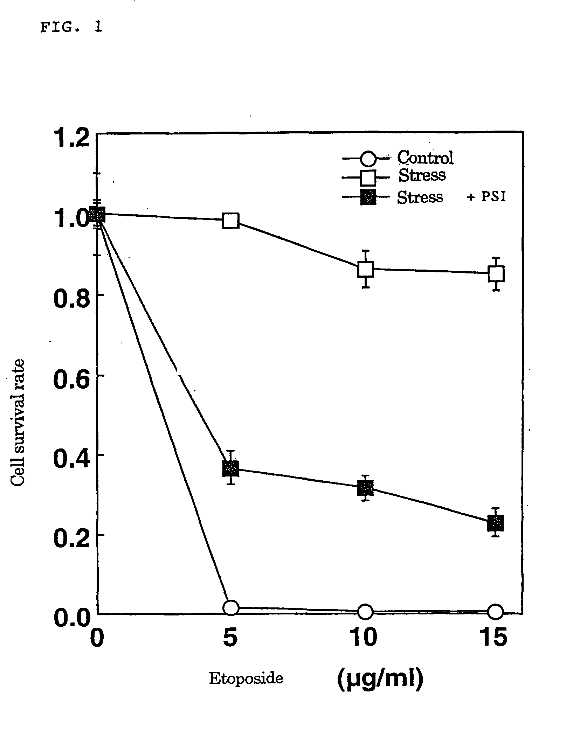

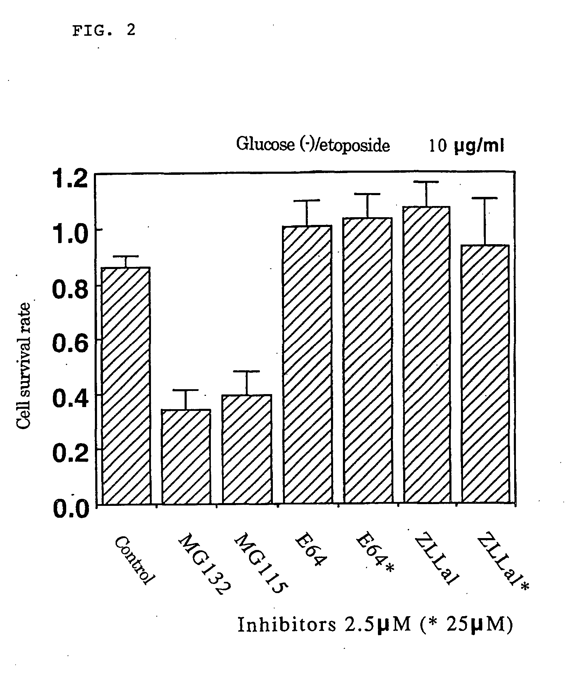

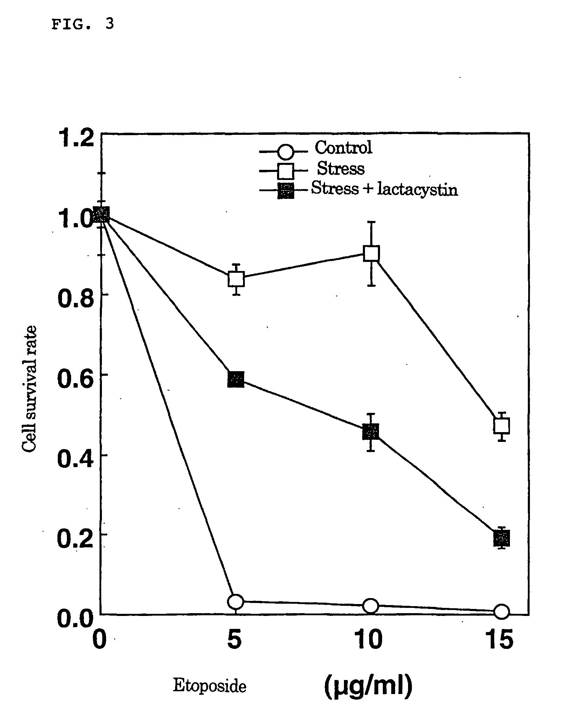

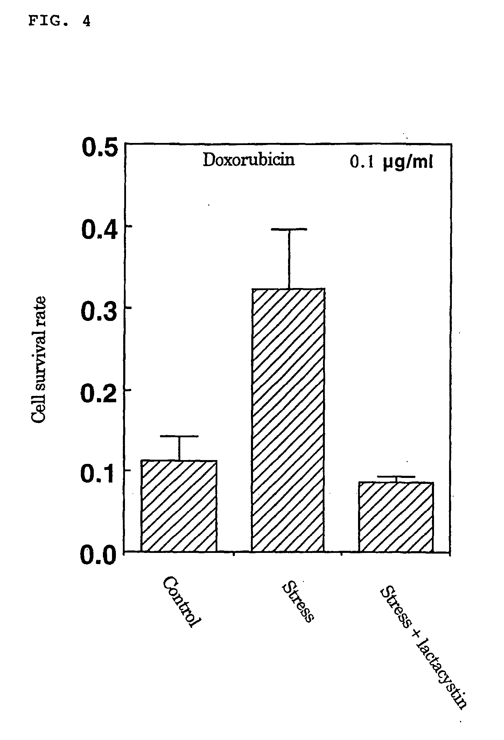

The result yielded will be presented in FIGS. 1 to 4. From the FIG. 1, it can be known that under the ordinary culture condition, in concentrations of 5, 10, and 15 µg/ml of the topoisomerase II-inhibiting anticancer drug, etoposide, almost all HT29 cells died and the colony cannot formed and that under the glucose starvation condition found in the solid cancer, cells showed resistance against etoposide to survive. In this occasion, it can be also known that if the proteasome inhibitor PSI presents in the culture medium, cells show sensitivity to etoposide even in the glucose starvation condition. From the FIG. 2, similarly, it can be also known that the proteasome inhibiting agent MG132 and MG115 enhance sensitivity of cells to etoposide (10 µg/ml) and the protease inhibiting agent E64 and ZLLal not inhibiting proteasome seldom change sensitivity to etoposide even using concentration 10 times the proteasome inhibiting agent. From FIG. 3, it can be also known that the proteasome inhibiting agent, lactacystin, having an action mechanism different from that of the above described inhibiting agent enhances sensitivity to etoposide. For reference, the difference in the action mechanism of the proteasome inhibiting agent is in that PSI, MG132, and MG115 inhibit proteasome reversibly and in contrast, lactacystin irreversibly inhibits by covalent bond with an active center of proteasome. In other words, it has been found that regardless of difference in inhibition mode, the proteasome inhibitor enhances sensitivity of etoposide, the topoisomerase II-inhibiting anticancer drug of cancer cells in the glucose starvation condition. In addition, from the FIG. 4, it has been found that sensitivity not only to etoposide, but also to doxorubicin expressing the anticancer action by inhibiting topoisomerase II are enhanced by the proteasome inhibiting agent.

### Example 2

### (Measurement of expression of topoisomerase IIα in the cultured cell line)

Topoisomerase II inhibiting anticancer drug stabilizes a complex (cleavable complex) consisting of the DNA and topoisomerase IIα occurring in a catalytic reaction process of topoisomerase IIα to cause DNA damage. Therefore, cell-killing effect of the topoisomerase II-inhibiting anticancer drug depends on the amount of intracellular expression of topoisomerase IIα essential for formation of the cleavable complex. Then, the amount of expression of topoisomerase IIα in the cultured cancer cell under the glucose starvation and hypoxia environment that are found in the solid cancer and the effect of the proteasome inhibiting agent to the amount of expression were examined.

The culture condition of cells is same as that described in Example 1 including the M phase synchronous culture method and culture under the glucose starvation condition. Culture under hypoxia condition, that is found in the solid cancer, similar to glucose starvation was carried out using the culture medium (that prepared by adding 5% FBS to RPMI1640 culture medium) under the ordinary condition in a Gaspack 100 anaerobic system (Becton Dickinson made). Culture under the glucose starvation condition and hypoxia condition was carried out for 14 hours . On the other hand, the proteasome inhibiting agent and the protease inhibiting agent not inhibiting proteasome were added to the culture medium in the final concentration of PSI: 5 µM, MG115: 5 µM, MG132: 5 µM, lactacystin: 10 µM, E64: 50 µM, and ZLLal: 50 µM, respectively.

With the purpose to prepare a whole cell extract solution, cells culture under the above described condition were washed with an ice-cooled PBS twice and then, cells were removed from the culture dish by using a cell scraper (Sumitomo Bakelite made) for collection. Cells collected were precipitated by centrifugation and then, dissolved in an SDS sample buffer to collect as the extract solution from all cells. For reference, the composition of the SDS sample buffer was 10% v/v, glycerol, 5% 2-mercaptoethanol, 2% SDS (sodium lauryl sulfate), and 62.5 mM tris-hydrochloric acid (pH 6.8).

The amount of topoisomerase IIα in the extract solution from all cells was measured by the western blotting method. The extract solution from all cells containing a total protein quantity of 30 µg was put on an SDS polyacrylamide gel (4-20 Multigel: Daiichi Pure Chemicals K.K. made) and subjected to electrophoresis in an electrophoresis buffer (25 mM tris, 192 mM glycine, and 0.1% SDS) in a 40 mA constant current for 1 hour. Subsequently, blotting was carried out in 4°C blotting buffer (25 mM tris, 192 mM glycine, and 20% methanol) in a 50 V constant voltage for 12 hours to transfer a protein separated in the SDS polyacrylamide gel to a nitrocellulose membrane. This nitrocellulose membrane was soaked in a blocking buffer (0.1% Tween 20, 4% skim milk-added PBS) for 1 hour to carry out blocking. The nitrocellulose membrane blocked was soaked in a solution, that was prepared by adding anti-topoisomerase IIα antibody (Cambridge Research Biochemicals Corp. made) of the final concentration 1 µg/ml as the primary antibody to the blocking buffer solution, for 1 hour. The nitrocellulose membrane was washed with the blocking buffer three times (5 minutes once), and then, soaked in the solution, in which a 1/1000 volume of an antimouse antibody (Amersham Pharmacia Biotech made) labeled with horse radish peroxidase as a secondary antibody had been added to the blocking buffer, for 1 hour. The nitrocellulose membrane was washed with the blocking buffer three times (5 minutes once), and then, washed with the PBS, to which 0.1% Tween 20 was added, three times (5 minutes once). The nitrocellulose membrane after completion of antibody reaction and washing was treated with the ECL reagent (Amersham Pharmacia Biotech made) after a handling manual and a topoisomerase IIα-specific signal was detected on an X-OMAT™AR film (Kodakmade). For reference, steps after blotting was carried out in a room temperature.

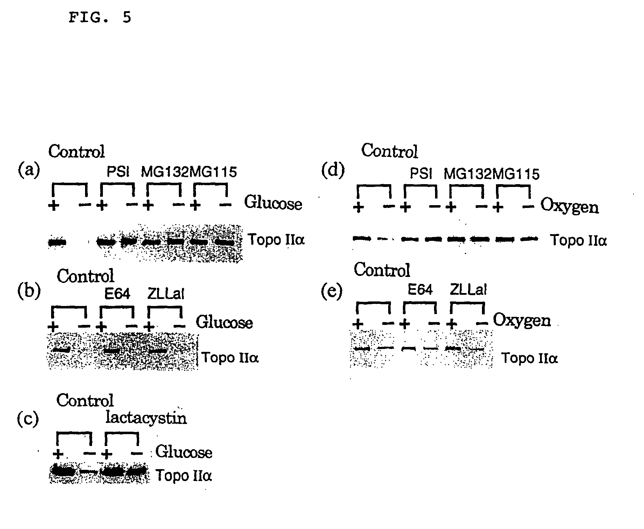

The result yielded will be shown in FIG. 5. As shown in control groups of FIGS. 5(a) to (c), it can be known that in cells in the glucose starvation condition, in comparison with cells in the ordinary culture condition, the expressed amount of topoisomerase IIα very dropped. This, as shown in Example 1, corresponds to cells in the glucose starvation condition present strong resistance against the anticancer drugs targeting topoisomerase II. On the other hand, from the FIG. 5(a), it can be known that in the presence of the proteasome inhibiting agent, PSI, MG132, and MG115, the expressed amount of topoisomerase IIα does not drop even in cells in the glucose starvation condition. Not presented as data, PSI, MG132, and MG115 inhibited similarly decrease in expression of topoisomerase IIα even in the final concentration of 2.5 µM. On the other hand, from the FIG. 5(c), it can be also known that the proteasome inhibiting agent, lactacystin, similarly inhibits drop of expression of topoisomerase IIα by the glucose starvation. However, as shown in FIG. 5(b), in the case of the protease inhibiting agent not inhibiting proteasome (E64 and ZLLal), the expressed amount of topoisomerase IIα very dropped. These results correspond highly to the result of enhancement of the effect of etoposide by the proteasome inhibitor of the example 1. In addition, from FIGS. 5 (d) and (e), it can be known that in hypoxia condition found in the solid cancer together with the glucose starvation condition, the proteasome inhibiting agent inhibits perfectly decrease in expression of topoisomerase IIα induced by hypoxia and the protease inhibiting agent not inhibiting proteasome presents no such effect. The above described results show that under the glucose starvation and hypoxia environments found in the solid cancer, the amount of expression of topoisomerase IIα decreases distinctly to show resistance to the anticancer drug, this decrease in expression is caused by enhancement of degradation of topoisomerase IIα, and proteasome involves in degradation of topoisomerase IIα.

### Example 3

### (Measurement of expression of proteasome in the cultured cell line)

Topoisomerase IIα is the protein located in the nuclei in cells. From the example 2, it was shown that topoisomerase IIα is degraded in the stressed environment found in the solid cancer and proteasome involves in this degradation. Then, whether proteasome presents actually in the nuclei of the HT29 cell and whether the amount of proteasome in the nuclei changes in the stressed environment were tested by employing immunological staining method and western blotting method.

For culture of the HT29 cells, the control group was that in which a 5% FBS culture medium was added to the ordinary RPMI1640 culture medium and for culture condition of the glucose starvation condition, that in which the 5% FBS culture medium was added to the ordinary RPMI1640 culture medium lacking glucose was employed. For culture under the hypoxia condition, that in which the 5% FBS culture medium was added to the RPMI1640 culture medium was employed. 4×10⁵ HT29 cells were inoculated in the 10 cm culture dish to culture in the ordinary culture medium (in which the 5% FBS was added to the RPMI1640 culture medium) for 2 days, washed with the PBS followed by replacing by the ordinary culture medium as the control group or the culture medium lacking glucose. The culture dish for the hypoxia condition was replaced by the ordinary culture medium followed by moving to the Gaspack 100 anaerobic system described in Example 2. Subsequently, it was cultured under the ordinary condition, glucose starvation condition, or hypoxia condition for 18 hours.

For immunological staining, the HT29 cells cultured under the condition as described above was fixed by soaking 10 minutes in the PBS to which 3.7% formaldehyde and 0.2% triton X100 were added. It was washed with the PBS three times and then, subjected to blocking in the PBS to which a 1% bovine serum albumin was added. This cell was reacted to the antibody (Progen Biotechnic made) against P27 of a proteasome subunit for 45 minutes. After washing with the PBS, it was reacted to a secondary antibody (antimouse antibody; Molecular Probe Co. made) bound to Oregon Green 488 for 30 minutes. In addition, after washing with the PBS, cells were soaked in propidium iodide solution (PI: Sigma-Aldrich made) of 50 µg/ml to stain intranuclear DNA. After washing with the PBS, cells stained were observed by using a confocal laser microscope (Leika made) to subjected photomicrography. Reactions as described above were all conducted at the room temperature.

In order to prepare a nuclear extraction solution for use in the western blotting method, the HT29 cells cultured under the above described condition were scraped off using the cell scraper as described in Example 2 for collection. The cells were washed once with a nuclear buffer (150 mM sodium chloride, 1 mM potassium dihydrogen phosphate, 5 mM magnesium chloride, 1 mM EGTA (ethylene glycol bis (β-aminoethyl ether)-N, N, N', N' tetra acetic acid), 1 mM dithiothreitol, 1 mM phenylmethylsulfonyl fluoride, 20 µg/ml Aprotinin, and 10% v/v glycerol; pH 6.4) and then, suspended again in the nuclear buffer to which 0.3% triton X-100 was added and put on ice for 10 minutes. Nuclei were collected by centrifugation in 450xg at 4°C for 10 minutes. The nuclei isolated were once washed with the nuclear buffer lacking Triton X-100, suspended again in the nuclear extraction buffer [20 mM HEPES-potassium hydroxide (pH 7.5), 0.1% CHAPS, 2mM EDTA, 5 mM dithiothreitol, 400 mM potassium chloride, 10 µg/ml leupepsin, 20 µg/ml pepstatin, 10 µg/ml Aprotinin, and 1 mM benzamidine], and rotated gently at 4°C for 1 hour. It was centrifuged at 15000×g at 4°C for 10 minutes to remove insoluble materials. The supernatant was dialyzed against an assay buffer [20 mM HEPES-potassium hydroxide (pH 7.5), 5 mM dithiothreitol, and 10% v/v glycerol] at 4°C for 2 hours. The supernatant, from which the insoluble materials were removed by centrifugation in 15000 x g, 4°C, and for 10 minutes, was collected as a nuclear extract fraction. The one-third volume of 4xsample buffer was added to the nuclear extract fraction to treat at 100°C for 5 minutes. On the other hand, the whole cell extract solution was collected by the method described in Example 2. Using the nuclear extract fraction and the whole cell extract solution, as described in Example 2, western blotting was carried out. For reference, as the SDS polyacrylamide gel for electrophoresis, a10-20 Multigel (Daiichi Pure Chemicals) was used and as the primary antibody, the antibody (Progen Biotechnic Corp. made) against a proteasome subunits P27 and P32 was used.

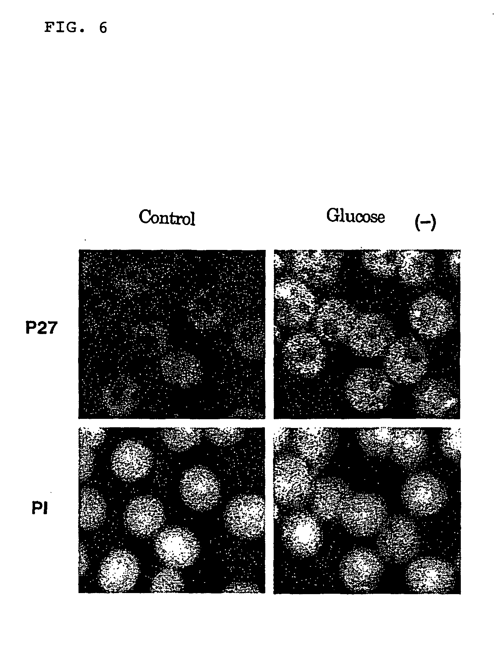

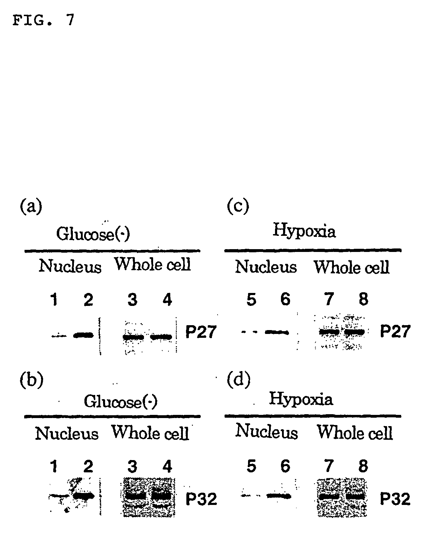

The result yielded will be presented in FIGS. 6 and 7. From the FIG. 6, it can be known that in cells in the glucose starvation condition, intensity of fluorescence in the nuclei stained immunologically was increased by the anti-P27 antigen and also proteasome in the nuclei increased. For reference, the bottom figure of the FIG. 6 shows the result of PI staining to stain DNA in a same field of view and a same section as those of the top figure and it can be known that both the control group and the glucose starvation condition were stained similarly. In other words, the figure shows that fluorescence presented in the top is of inside the nuclei. From the western blotting analysis shown in the FIG. 7(a), it was found that the amount of expression of the proteasome subunit P27 in the nuclear fraction increased in the glucose starvation condition of a lane 2 in contrast to the control group of the lane 1. On the other hand, it can be known that the amount of expression of P27 of the whole cell extract solution is same as that of the control group in the glucose starvation condition of the lane 4 in contrast to the control group of the lane 3. Similarly, from the FIG. 7 (b), it can be known that the amount of expression of P32, which is another proteasome subunit, also increased in the glucose starvation condition of the lane 2 in comparison with the control group of the lane 1 in the nuclear fraction and in contrast, in the whole cell extract solution, there is no change between the control group of the lane 3 and the glucose starvation condition of the lane 4. From analysis by densitometry, it was found that the amount of expression of the P27 and P32 in the nuclear fraction increased about three times in the glucose starvation condition. Similarly, it can be known that in cells in the hypoxia condition, the expression amount of P27 in the nuclear fraction increased in the hypoxia condition of the lane 6 in comparison with the control group of the lane 5 of the FIG. 7 (c) and the amount of expression of P32 in the nuclear fraction increased in the hypoxia condition of the lane 6 in comparison with the control group of the lane 5 of the FIG. 7 (d). From the result of the densitometry analysis, it was found that these increases are about twice. In FIGS. 7 (c) and (d), it can be known that the lane 7 and lane 8 show the expression amounts of P27 and P32 in the whole cell extract solution in the control group and the hypoxia condition, respectively; however, both did not change in the control group and the hypoxia condition. Consequently, it was shown that proteasome presents in the nuclei in the glucose starvation and the hypoxia conditions and the expression amount in the nuclei increases in comparison with the ordinary culture condition. The expression amount of the subunit in the whole cell extract solution is not different from cells under the ordinary culture condition and hence, it can be known that proteasome is accumulated in the nuclei under the stressed environment. This does not conflict with that proteasome involves in degradation and enhancement of topoisomerase IIα localized in the nuclei under that stressed environment shown in Example 2. On the other hand, in the case where glucose starvation is compared with the hypoxia condition in Example 2, the glucose starvation condition shows the larger decrease in expression of topoisomerase IIα. This fact well coincides with that an increased amount of proteasome in the nuclei in larger in the glucose starvation condition.

### Example 4

### (Measurement of proteasome activity in non-cultured cell line)

Whether proteasome appearing in the nuclei in the cell has an actual activity or whether the activity is suppressed by the proteasome inhibitor were examined.

Culture of the HT29 cell and collection of the nuclear extract solution were carried out as described in the example 3. Proteasome activity in the nuclear extract solution was examined using a fluorescent peptide substrate. A nuclear protein 10 to 40 µg extracted into 25 µl of the assay buffer [20 mM HEPES-potassium hydroxide (pH 7.5, 5 mM dithiothreitol, and 10% v/v glycerol] was mixed with the fluorescent peptide substrate of the final concentration 20 µM to react at 37°C for 1 hour. 10 µl of 10% SDS was added to this reaction mixture to stop the reaction. When the effect of the proteasome inhibiting agent and the protease inhibiting agent not inhibiting proteasome were examined, the nuclear protein and the inhibiting agent were previously mixed to react at 37°C for 10 minutes and then, the fluorescent peptide substrate was added to the reaction solution. After stop of the reaction, 450 µl pure water was added to the reaction mixture solution to measure intensity of fluorescence at 460 nm in excitation wavelength of 380 nm by using a fluorescence spectrophotometer (Hitachi Ltd. made). This fluorescent intensity was assigned to proteasome activity in the nuclear extract solution.

The proteasome inhibiting agent and the protease inhibiting agent not inhibiting proteasome which were sued in the example are those described in Example 1. The final concentrations used were 2.5 µm for the proteasome inhibiting agents PSI, MG115, and MG132, 5 and 10 µm for E64, and 2.5 and 5 µm for ZLLal, respectively. The fluorescent peptide substrates used were those as follows.

### Z-LLL-MCA

(carbobenzoxy-L-Leucyl-L-Leucyl-L-Leucine 4-methyl-coumaryl-7-amide): Peptide Institute Inc. made

### Suc-LLVY-MCA

(succinyl-L-Leucyl-L-Leucyl-L-Valyl-L-Tyrosine-4-meth yl-coumaryl-7-amide): Peptide Institute Inc. made

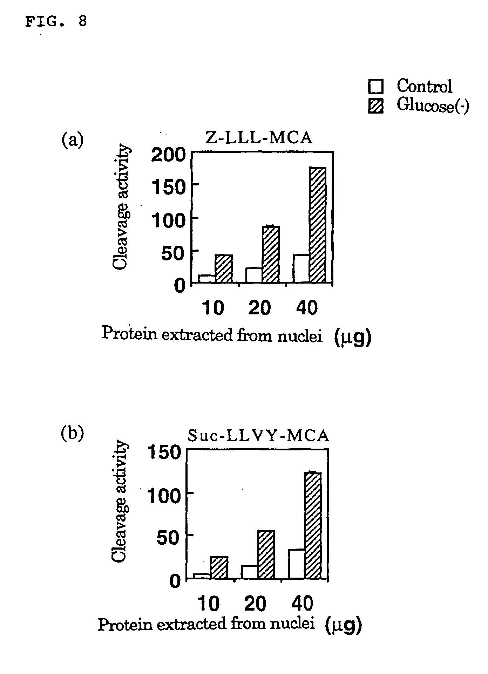

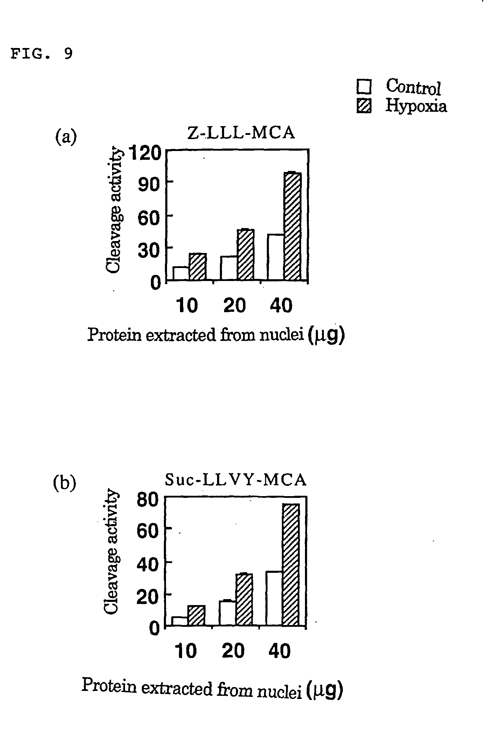

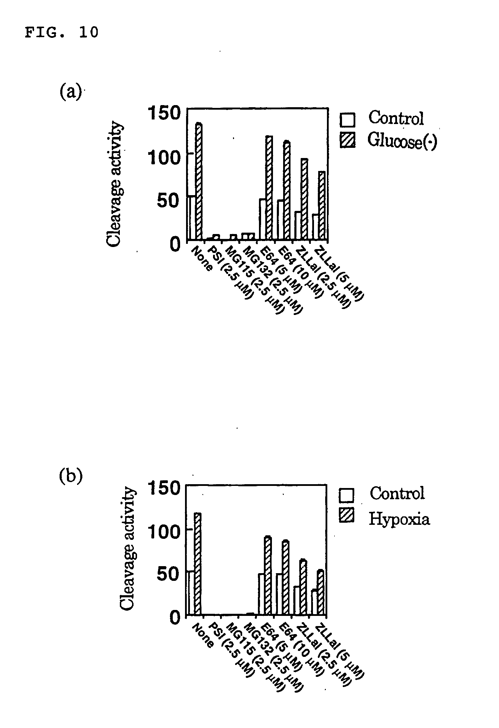

The result yielded will be shown in FIGS. 8 to 10. FIG. 8(a) and FIG. 8(b) show the results of measurement of proteasome activity in the nuclear extract solution by using Z-LLL-MCA as the substrate and Suc-LLVY-MCA, respectively, as the substrate. It can be known that either substrate used showed a proportional proteasome activity on a total nuclear protein amount added to the reaction solution and cells in the glucose starvation condition is higher in the proteasome activity in the nuclear extract solution. From the FIG. 9, it can be known that also in cells in hypoxia, the proteasome activity is higher in the nuclear extract solution. For reference, FIG. 9 (a) and FIG. 9 (b) show the results of measurement in use of Z-LLL-MCA and Suc-LLVY-MCA, respectively, as the substrate. Quantitatively, it was found that in the glucose starvation, Z-LLL-MCA showed 4.2 times higher and in Suc-LLVY-MCA, 3.7 times higher result and on the other hand, in case of hypoxia, Z-LLL-MCA showed 2.4 times higher and in Suc-LLVY-MCA, 2.2 times higher result were yielded, respectively. This quite coincident to the result of the example 4 and shows that accumulation of proteasome in the nuclei in the stress involves directly in elevation of proteasome activity in the nuclei. In addition, from FIG. 10, it can be known that in case using Z-LLL-MCA as the substrate, the proteasome inhibiting agent (PSI,MG115, and MG132) can suppress almost perfectly proteasome activity in the nuclear extract solution. On the other hand, the protease inhibiting agent not inhibiting proteasome can hardly suppress proteasome activity. For reference, FIG. 10 (a) shows the result in case using the nuclear extract solution of cells of the control group and the glucose starvation condition and FIG. 10 (b) shows the result in case using the nuclear extract solution of cells of the control group and the hypoxia condition.

### Example 5

### (Enhancement of the effect of anticancer therapy in a human cancer cell-transplanted mouse model 1)

A human colon cancer cell HT29 was transplanted subcutaneously to a nude mouse to examine a therapeutic effect of combined use of lactacystin, the proteasome inhibiting agent, with etoposide, the anticancer drug targeting topoisomerase II.

As an experimental animal, the nude mouse BALB/cAnNCrj-nu/nu of 8-week old female (Charles River Laboratories product) was used. In order to form a tumor lump in the nude mouse, 1×10⁷ HT29 cells was subcutaneously transplanted. After 8 to 10 days of transplanting, the tumor lump of HT29 cells grew to 100 to 150 m³ and at this point, the therapy was started.

Etoposide and lactacystin used were those prepared in a drug as the anticancer drug the agent for enhancing the effect of the anticancer drug. In other words, etoposide was used as an injection prepared by dissolving in water, which contain Japanese Pharmacopoeia Polysorbate 80 (80 mg/ml), Macrogol 300 (650 mg/ml), absolute ethanol (a proper volume), and Japanese Pharmacopoeia benzyl alcohol (30 mg/ml) as additives to make 20 mg/ml and lactacystin was used as the injection by dissolving in saline to make 100 mg/ml.

The therapy was conducted by dividing the HT29 tumor-transplanted mice into 4 groups to assign 4 animals to 1 group as follows by administering intraperitoneally three times every 4 days: (1) control group untreated with saline, (2) etoposide 33 mg/kg, (3) lactacystin 40 mg/kg, (4) lactacystin 40 mg/kg and etoposide 33 mg/kg. For reference, in case of (4) administration in combined use of lactacystin and etoposide, lactacystin was previously administered and then after 5 hours, etoposide was administered. Since start of the therapy, a size of the tumor lump was measured to measure effect of the therapy by administration of drugs once every 4 days.

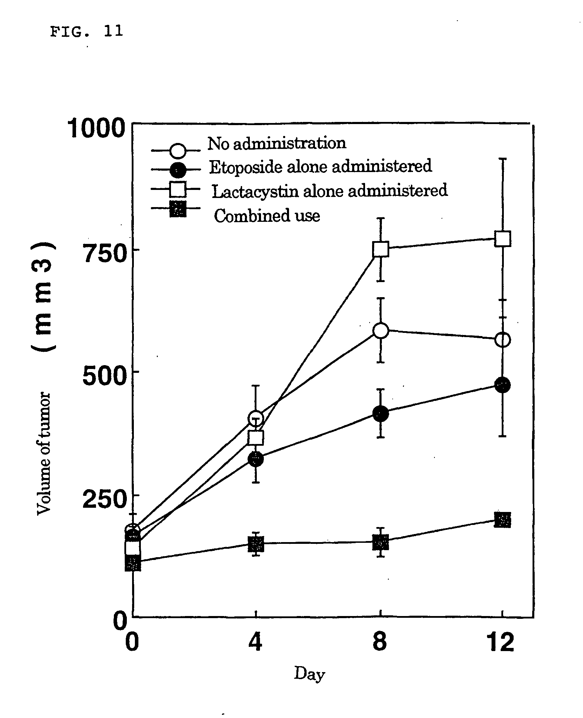

The result yielded will be shown in FIG. 11. From the FIG. 11, in comparison with the untreated group, therapies by administration of lactacystin alone and etoposide alone showed less suppression of tumor proliferation, that is an index of the antitumor effect. On the other hand, the therapy by combined use of lactacystin and etoposide showed almost no proliferation of the tumor immediately after the start of the therapy and hence, it can be known that combined use of lactacystin and etoposide presents a strong anticancer effect.

### Example 6

### (Enhancement of the effect of anticancer therapy in a human cancer cell-transplanted mouse model 2)

Similar to the above described example 5, the therapeutic effect of simultaneous administration of both drugs, that are lactacystin, the proteasome inhibiting agent, and etoposide, topoisomerase II-targeting anticancer drug, was examined. Transplantation of the HT29 cell to the nude mouse was carried out as described in the example 5. The therapy was started at the point where the tumor lump of the HT29 cell reaches a size ranging from 100 to 150 mm³. The HT29 tumor-transplanted mice were divided into 4 groups to assign 6 animals to 1 group. The therapy was conducted twice every 4 days by intraperitoneal dministration of the drugs as described below for 1 frequency. (1) Saline was administered for the untreated control group, (2) etoposide of 33 mg/kg was administration, (3) lactacystin of 25 mg/kg was administered, and (4) lactacystin of 25 mg/kg and etoposide of 33 mg/kg were simultaneously administered. Once every 4 days since start of the therapy, the size of the tumor lump was measured to measure the therapeutic effect by drug administration.

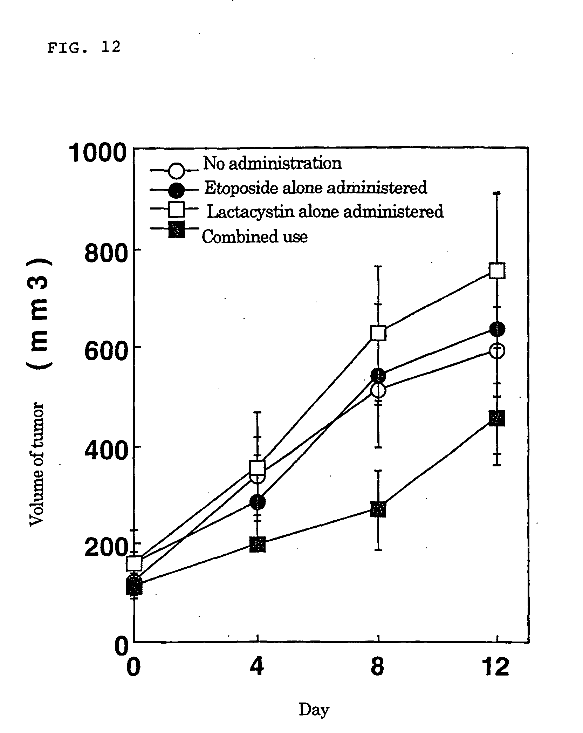

The result yielded will be shown in FIG. 12. From the FIG. 12, it can be known that in contrast to the untreated group and treated groups by the administration of lactacystin alone or etoposide alone, the therapy by combined use of lactacystin and etoposide presents a strong antitumor effect. In other words, it was found that the combined use of the proteasome inhibiting agent, lactacystin, and the topoisomerase II-targeting anticancer, etoposide, presents the stronger antitumor effect than the therapy by them alone in simultaneous administration.

### Example 7

### (Enhancement of the effect of anticancer therapy in a human cancer cell-transplanted mouse model 3)

The effect of therapy by simultaneous administration of the proteasome inhibiting agent, lactacystin, and the topoisomerase II-targeting anticancer, doxorubicin was examined. Transplantation of the HT29 cell to the nude mouse was carried out as described in the example 5. The treatment was started at the point where the tumor lump of the HT29 cell reached a range from 100 to 150 mm³. The HT29 tumor-transplanted mice were divided into 4 groups to assign 6 animals to 1 group. The treatment was conducted twice every 4 days by intraperitoneal administration of the drug as follows for 1 frequency. (1) Untreated control group, received saline, (2) doxorubicin of 6 mg/kg was administered, (3) lactacystin of 33 mg/kg was administered, (4) simultaneous administration of lactacystin of 33 mg/kg, and doxorubicin of 6 mg/kg. After start of treatment, once every 4 to 5 days, the size of the tumor lump was measured to measure the effect of treatment by drug administration.

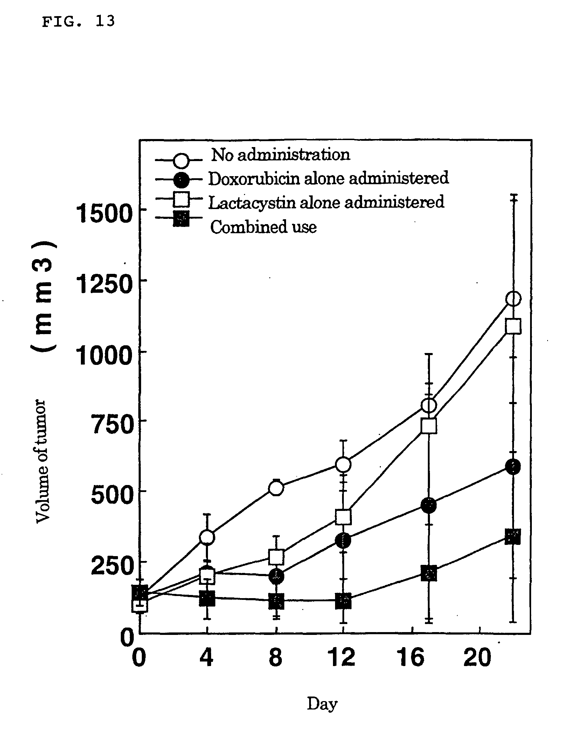

The result yielded will be presented in FIG. 13. From the FIG. 13, it can be known that the stronger anticancer effect is yielded by the treatment by combined use of lactacystin and doxorubicin than the untreated group, the treated groups by the administration of lactacystin alone, or administration of doxorubicin alone. In other words, the proteasome inhibitor, lactacystin, in combined use not only with etoposide, but also with the anticancer drug, doxorubicin, targeting similarly topoisomerase II, shows the stronger anticancer effect than that of the treatment alone, respectively.

### Example 8

### (Enhancement of the effect of the topoisomerase I-targeting anticancer drug)

The examples as described above showed the proteasome inhibiting agent enhances the effect of the topoisomerase II targeting anticancer drug on a cell culture level and the effect is reproduced on an animal level. Thus, to examine whether the proteasome inhibiting agent has the activity on enhance the effect of the anticancer drug other than the topoisomerase II-targeting anticancer drug, the effect of combined use of the proteasome inhibiting agent with the topoisomerase I inhibitor, camptothecin, was examined using the cultured cell line. Camptothecin is a mother compound of the anticancer drug, CPT-11, clinically used currently, and it can be known these antitumor effect are caused by inhibition of topoisomerase I, different from etoposide and doxorubicin.

The cell culture condition for both M
phase-synchronized culture method and culture under the glucose starvation condition was as described in Example 1. M phase-synchronized cells were cultured in the culture medium, to which lactacystin of the final concentration 10 µM was added, or the culture medium, to which this was not added, as the proteasome inhibiting agent under the ordinary condition and the glucose starvation condition, respectively, for 13 hours. In case of the proteasome inhibiting agents, PSI and MG132, M phase-synchronized cells were cultured in the culture medium, to which these of the final concentration 2.5 µM was added or the culture medium, to which these are not added, respectively, under the glucose starvation condition for 13 hours. In order to examine sensitivity of cells against the topoisomerase I inhibiting agent, camptothecin (Yakult made), the drug was added to the culture medium to continue further culturing for 4 hours. Sensitivity of cells was measured by applying the colony formation method described in Example 1. On the other hand, the amount of expression of topoisomerase I that is the target molecule of camptothecin was measured by preparing the whole cell extract solution as described in Example 2 followed by the western blotting method. For reference, in measuring the amount of expression of topoisomerase I, regarding the proteasome inhibiting agents, clasto-lactacystin β lactone (Boston Biochem made), which is an activated form of lactacystin, was used in the final concentration of 2.5, 5, and 10 µM. Meanwhile, as the antibody against human topoisomerase I, a mouse monoclonal antibody established by Tsuruo et al. was used.

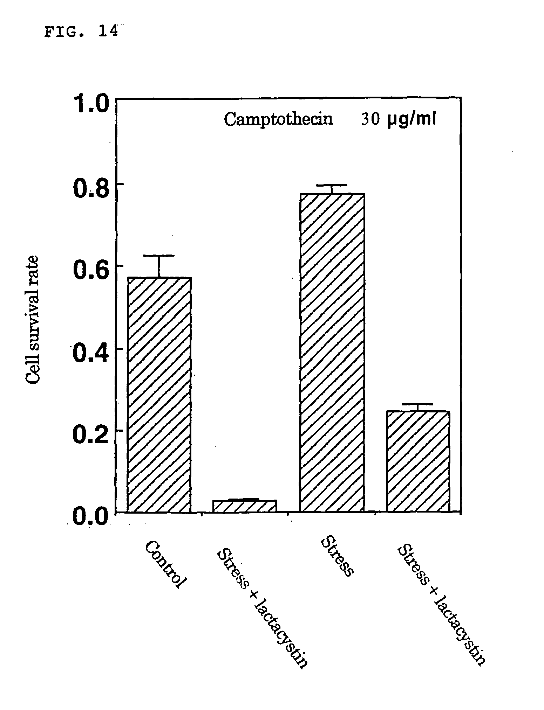

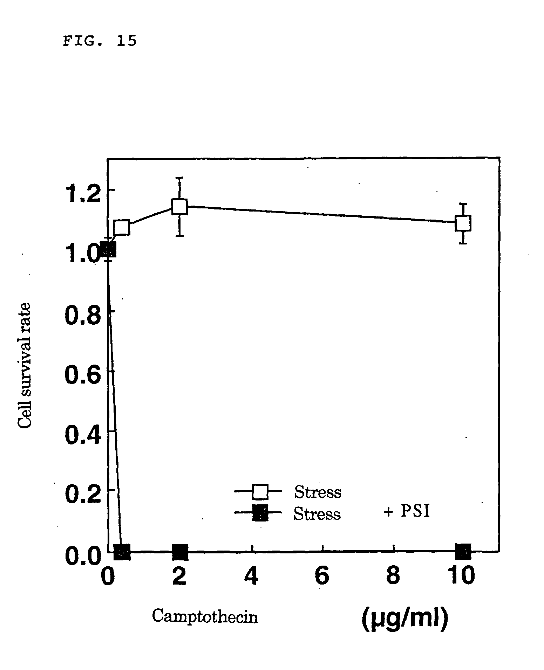

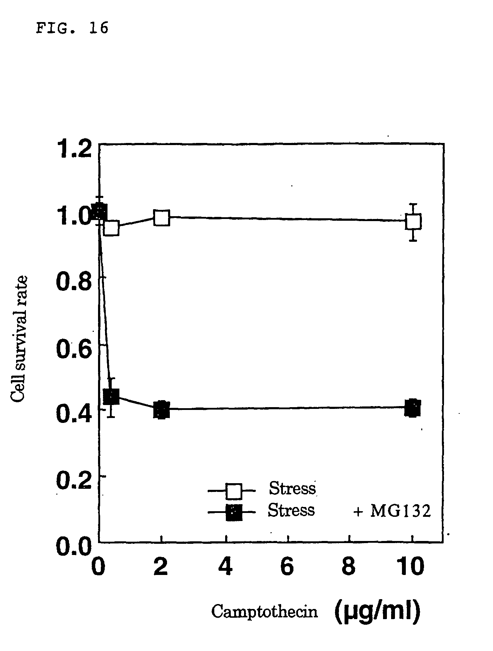

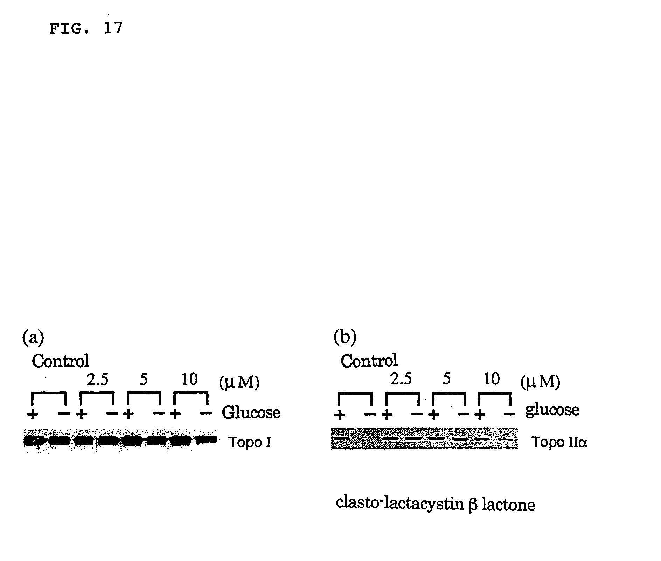

The result yielded will be presented in FIGS. 14 to 17. From the FIG. 14, similar to case of the topoisomerase II-targeting anticancer drug described in Example 1, it can be known that lactacystin as the proteasome inhibiting agents enhances the cell-killing effect of camptothecin as the target of topoisomerase I under the glucose starvation condition found in the solid cancer. Interestingly, it can be known that in case of camptothecin, the cell-killing effect thereof is enhanced by lactacystin also under the ordinary culture condition. In other words, enhancement of the effect of camptothecin by lactacystin as the proteasome inhibiting agents is unrestrictedly observed in the glucose starvation condition. From the FIGS. 15 and 16, it can be known that the proteasome inhibiting agents, PSI and MG132, similar to camptothecin, also enhance the effect of camptothecin. In other words, it can be known that for enhancement of the effect of camptothecin, the proteasome inhibiting activity is important. Camptothecin stabilizes the cleavable complex of topoisomerase I and DNA to induce death of cells and thus, it has been known that similar to case of the topoisomerase II-targeting anticancer drug, the cell-killing effect thereof correlates with the amount of expression of topoisomerase I, that is the target molecule. However, from FIG. 17(a), it can be known that the amount of expression of topoisomerase I does not change, as shown in the control group, between the ordinary condition and the glucose starvation condition and does not change regardless of addition of the activated type of lactacystin (2.5, 5, and 10 µM). For reference, FIG. 17(b) shows the result of examination of the amount of expression of topoisomerase IIα by using the same cell extract solution as that of FIG. 17(a) and it can be known that from the two lanes of the control group, the amount of expression of topoisomerase IIα lowered under the glucose starvation condition and also it can be known that the lowering of expression is perfectly inhibited by adding 2.5, 5, and 10 µM of the activated type of lactacystin. Therefore, enhancement of the effect of the topoisomerase I-targeting anticancer drug by the proteasome inhibiting agents, differing from case of the topoisomerase II-targeting anticancer drug, depends on unknown mechanisms not accompanying the change of the amount of expression of topoisomerase I. Concerning this unknown mechanisms, on the basis of the fact that proteasome is the enzyme performing degradation of intracellular protein and hence, it is presumed that the enzyme degrades an important protein for expression of the cell-killing effect of the topoisomerase I-targeting anticancer drug to lead to resistance to the anticancer drug. It is presumed that the proteasome inhibiting agents inhibits this degradation to enhance the effect of the topoisomerase I-targeting anticancer drug.

### Example 9

### (Enhancement of the effect of cisplatin in the cultured cell line)

It has become evident that from all examples as described above, the proteasome inhibitor enhances the effect of the topoisomerase I-and II-targeting anticancer drugs. In addition, in order to examine whether the proteasome inhibitor enhances the effect of cisplatin, the effect of combined use of lactacystin as the proteasome inhibiting agents and cisplatin was examined using the cultured cell line.

The culture condition of cells for both M phase-synchronized culture method and culture under the glucose starvation condition were those as described in the Example 1. M phase-synchronized HT29 cells were cultured in the culture medium, to which lactacystin of the final concentration 7.5 µM was added, or the culture medium, to which this was not added, as the proteasome inhibiting agent under the ordinary condition and the glucose starvation condition, respectively, for 13 hours. In order to examine sensitivity of cells against cisplatin (Bristol Myerss Squibb K.K. made), the drug was added to each culture medium and the cells were further cultured for 4 hours . Sensitivity of cells was measured by applying the colony formation method described in Example 1.

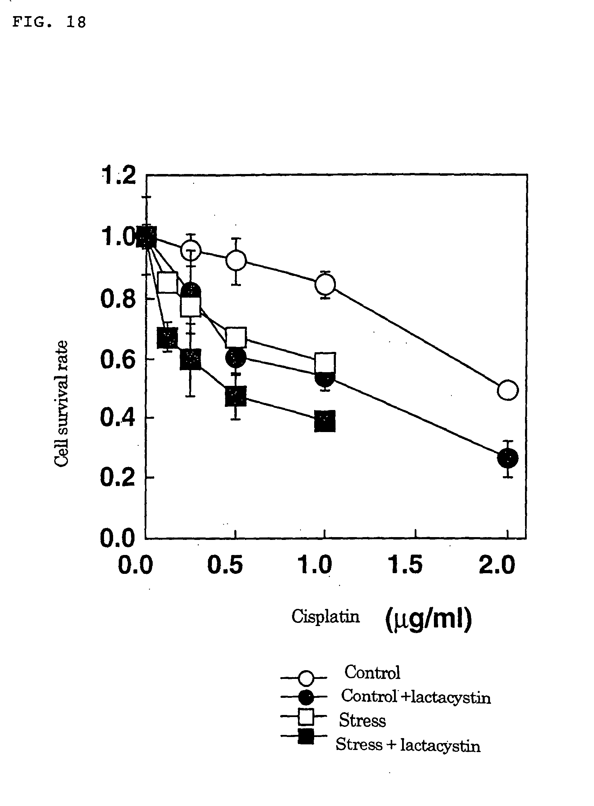

The result yielded will be presented in FIG. 18. From the FIG. 18, it has become evident that lactacystin as the proteasome inhibiting agent enhances the cell-killing effect of cisplatin. It can be known that enhancement of the effect of cisplatin by lactacystin is, similar to case of the topoisomerase I-targeting anticancer drug, effective under any one of the ordinary culture condition and the glucose starvation condition. By the way, in difference from the topoisomerase targeting anticancer drug, cancer cells showed a high sensitivity to cisplatin under the stress environment and it is interesting that this fact coincides with that cisplatin shows the highest efficiency in current clinical trials. Further effect enhancement by lactacystin as the proteasome inhibitor is expected to contribute to improve the result of treatment of the solid cancer by cisplatin. It can be presumed that cisplatin binds to the DNA strand in cancer cells to inhibit DNA synthesis and subsequent division of cancer cells. However, it has not been evident at present how the proteasome inhibiting agent induces enhancement of cisplatin's effect.

### Example 10

### (Enhancement of the effect of etoposide by a lactacystin derivative in the cultured cell line)

Whether decarboxy lactacystin, that is a derivative of lactacystin, induces enhancement of the effect of etoposide, the topoisomerase II-targeting anticancer drug, was examined.

The cell culture condition for both M
phase-synchronized culture method and culture under the glucose starvation condition was as described in Example 1. M phase-synchronized HT29 cells were cultured in the culture medium, to which decarboxy lactacystin of the final concentration 5 µM was added, or the culture medium, to which this was not added, as the proteasome inhibiting agent under the ordinary condition and the glucose starvation condition, respectively, for 13 hours. In order to examine sensitivity of cells to etoposide (Bristol Myerss Squibb K. K. made), the drug was added to each culture medium and the cells were further cultured for 4 hours. Sensitivity of cells was measured by applying the colony formation method described in Example 1. On the other hand, the M phase-synchronized HT29 cells were cultured in the culture medium, to which decarboxy lactacystin of the final concentration 0.5, 1, 2.5, and 5 µM was added, or the culture medium, to which these were not added, as the proteasome inhibiting agent under the ordinary condition and the glucose starvation condition for 13 hours to measure the amount of expression of topoisomerase IIα, that is the target molecule of etoposide. By the way, the amount of expression of topoisomerase IIα was measured by preparing the whole cell extract solution followed the western blotting method, as described in Example 2.

The result yielded will be presented in FIG. 19. From the top of the FIG. 19, it can be known that decarboxy lactacystin as the proteasome inhibitor, similar to lactacystin, enhances the cell-killing effect of etoposide. From the bottom of the FIG. 19, it can be known that decarboxy lactacystin as the proteasome inhibitor, similar to lactacystin, inhibits lowering of expression of topoisomerase IIα by glucose starvation.

### Example 11

### (Enhancement of the effect of SN38 by the lactacystin derivative in the cultured cell line)

Next, whether decarboxy lactacystin, that is the derivative of lactacystin, induces enhancement of the effect of SN38, the topoisomerase I-targeting anticancer drug, was examined. For reference, SN38 is the derivative of camptothecin and the active form of Irinotecan (CAT-11), that is being used clinically.

The cell culture condition was as described in Example 1. M phase-synchronized HT29 cells were cultured in the culture medium, to which decarboxy lactacystin of the final concentration 5 µM was added, or the culture medium, to which this was not added, as the proteasome inhibiting agent under the ordinary condition, respectively, for 13 hours. In order to examine sensitivity of cells to SN38 (Yakult made), the drug was added to each culture medium and the cells were further cultured for 4 hours. Sensitivity of cells was measured by applying the colony formation method described in Example 1.

The result yielded will be presented in FIG. 20. From the FIG. 20, similar to lactacystin, it has been evident that decarboxy lactacystin, which is the derivative of lactacystin, enhances the cell-killing effect of SN38, the derivative of camptothecin, under the ordinary condition.

### Example 12

### (Enhancement of the effect of mitomycin C and bleomycin in the cultured cell line)

In order to examine whether the proteasome inhibiting agent enhances the effect of alkylating agents and bleomycins, the effect of combined use of lactacystin as the proteasome inhibiting agent, mitomycin C, and bleomycin was examined in the cultured cell line.

The cell culture condition for both M
pahse-synchronized culture method and culture under the glucose starvation condition was as described in Example 1. Mphase-synchronized cells were cultured in the culture medium, to which lactacystin of the final concentration 7.5 µM was added, or the culture medium, to which this was not added, as the proteasome inhibiting agent under the glucose starvation condition, respectively, for 13 hours. In order to examine sensitivity of cells to mitomycin C (Kyowa Hakko made) and bleomycin (Nippon Kayaku made), the drug was added to each culture medium and the cells were further cultured for 4 hours. Sensitivity of cells was measured by applying the colony formation method described in Example 1.

The result yielded will be presented in FIG. 12. From the FIG. 12, it has been evident that lactacystin, that is the proteasome inhibiting agent, enhances the cell-killing effect of mitomycin C (a) and bleomycin (b) under the glucose starvation condition.

For reference, mitomycin C was prepared as the injection one of drug forms for the anticancer drug by adding Japanese Pharmacopoeia water for injection in a proportion of 5 ml to 2 mg of mitomycin C (factor). This was diluted to the predetermined concentration, when required, to use for the above described operation.

On the other hand, bleomycin of 15 mg to 30 mg (factor) as bleomycin hydrochloride was dissolved in saline of about 5 to 20 ml to prepare injection, one of drug forms as the anticancer drug. This was diluted to the predetermined concentration, when required, to use for the above described operation.

As described above, it can be known that against cancer cells exposed to the physiological stress inside the solid cancer and tolerated to the topoisomerase II-targeting anticancer drug; the proteasome inhibiting agent shows very rationally efficiency as the agent to enhance the effect of the anticancer drug. In addition, as shown in Example 8 using the topoisomerase I-targeting anticancer drug, Example 9 using cisplatin being the platinum complex, and Example 12 mitomycins being the alkylating agent, and bleomycin being bleomycins, even any anticancer drug, in the case where it can be presumed that the proteasome degrades the important protein for expression of the cell-killing effect by the anticancer drug to lead to resistance against the anticancer drug, regardless of the environment where cancer cells located in, it is distinctly evident that the proteasome inhibiting agent can be used as the agent to enhance the effect of the anticancer drug.

## Claims

1. An anticancer drug enhancer, to enhance an effect of an anticancer drug, **characterized in that** an effective ingredient is a substance inhibiting the activity of proteasome having a function to enhance an effect of a DNA strand-damaging type anticancer drug to cancer cells.

2. The anticancer drug enhancer according to claim 1, **characterized in that** said DNA strand-damaging type anticancer drug is an anticancer drug whose effect is suppressed or reduced in the presence of an activity of proteasome.

3. The anticancer drug enhancer according to claim 1, **characterized in that** said anticancer drug contains a substance inhibiting an activity of topoisomerase as the effective ingredient.

4. The anticancer drug enhancer according to claim 2, **characterized in that** the anticancer drug is at least one of a substance inhibiting an activity of topoisomerase I and a substance inhibiting an activity of topoisomerase II.

5. The anticancer drug enhancer according to claim 1, **characterized in that** said DNA strand-damaging type anticancer drug is an anticancer drug damaging the DNA by acting directly to DNA.

6. The anticancer drug enhancer according to any one of claim 1 to claim 5, **characterized in that** said substance inhibiting the activity of proteasome is a selective proteasome activity inhibiting agent.

7. The anticancer drug enhancer according to any one of claim 1 to claim 5, **characterized in that** said substance inhibiting the activity of proteasome is a non-peptide aldehyde proteasome activity inhibiting agent.

8. The anticancer drug enhancer according to any one of claim 1 to claim 7, **characterized in that** said cancer is a solid cancer.

9. The anticancer drug enhancer according to any one of claim 1 to claim 7. **characterized in that** said cancer is under a physiological stress.

10. The anticancer drug enhancer according to claim 9, **characterized in that** said physiological stress is glucose starvation stress or hypoxia stress.

11. A drug set of an anticancer drug and an enhancer to enhance an effect of the anticancer drug, **characterized in that** said enhancer is a substance inhibiting an activity of proteasome which has a function to enhance the effect of said anticancer drug by inhibiting the activity of proteasome in a cancer cell and
said anticancer drug is an anticancer drug whose effect can be suppressed or reduced the effect thereof in the presence of the proteasome activity.

12. The drug set according to claim 11, **characterized in that** said anticancer drug contains the substance inhibiting the activity of topoisomerase as the effective ingredient.

13. The drug set according to claim 12, **characterized in that** the anticancer drug is at least one of a substance inhibiting an activity of topoisomerase I and a substance inhibiting an activity of topoisomerase II.

14. The drug set according to claim 11, **characterized in that** said substance inhibiting an activity of proteasome is lactacystin.

15. The drug set, according to any one of claim 11 to claim 14, being a drug set of 2 preparations comprising a preparation containing said anticancer drug and a preparation containing other said substance inhibiting an activity of proteasome.

16. The drug set according to any one of claim 11 to claim 14, having a mixture form containing said anticancer drug and said substance inhibiting the activity of proteasome in a same preparation.

17. The drug set according to any one of claim 11 to claim 16, **characterized in that** said cancer is a solid cancer.

18. The drug set according to any one of claim 11 to claim 16, **characterized in that** said cancer is under physiological stress.

19. The drug set according to claim 18, **characterized in that** said physiological stress is glucose starvation stress or hypoxia stress.

20. A drug set of an anticancer drug damaging DNA by acting directly to the DNA and an agent inhibiting an activity of proteasome.

21. The drug set according to claim 20, **characterized in that** said anticancer drug damaging the DNA by acting directly to DNA is platinum complexes, alkylating agents, and bleomycins.

22. The drug set according to claim 20, **characterized in that** said substance inhibiting an activity of proteasome is a selective proteasome activity inhibiting agent.

23. The drug set according to claim 22, **characterized in that** said selective proteasome activity inhibiting agent is lactacystin.

24. A therapeutic method for cancer **characterized in that** a DNA strand-damaging type anticancer drug and a substance inhibiting the activity of proteasome, that has a function to enhance the effect of the DNA strand-damaging type anticancer drug against cancer cells, is administered.

25. Use of a substance inhibiting an activity of proteasome and having a function to enhance an effect of a DNA strand-damaging type anticancer drug to cancer cells for manufacturing the agent to enhance the effect of the anticancer drug.
